# EUROPEAN PATENT APPLICATION

(11) **EP 1 726 955 A1**
(43) Date of publication of application: **29.11.2006**
(21) Application number: 05720894.4
(22) Date of filing: 16.03.2005
(51) Int. Cl.: G01N 33/52, G01N 21/78, C12Q 1/28

(54) **METHOD OF STABILIZING OXIDIZABLE COLOR-ASSUMING REAGENT**

(30) Priority: 17.03.2004 JP 2004076015
(71) Applicant: DAIICHI PURE CHEMICALS CO., LTD., Chuo-ku Tokyo 103-0027 (JP)
(72) Inventor: TANIGUCHI, Yuriko; C/O DAIICHI PURE CHEMICALS CO.,, Ibaraki;3 (JP); NISHIO, Tomohisa; C/O DAIICHI PURE CHEMICALS CO.,, RYUGASAKI-SHI, Ibaraki;30 (JP); SAITO, Kazunori; C/O DAIICHI PURE CHEMICALS CO., L, RYUGASAKI-SHI, Ibaraki;301 (JP)
(74) Representative: Hartz, Nikolai
(86) International application number: PCT/JP2005/004640
(87) International publication number: WO 2005/088305

(57) **Abstract**

The invention provides a method for stabilizing an oxidizable color developing reagent, in particular, a leuco dye, as well as a stabilizing reagent employed therefor. Specifically, the invention is directed to a method for stabilizing an oxidizable color developing reagent, which is attained by storing the reagent in a solution of pH 1 to 5; and to a stabilizing oxidizable color developing reagent.

## Description

### Technical Field

The present invention relates to a method for stabilizing an oxidizable color developing reagent to be used for assaying minor components of a biological sample, and to an oxidizable color developing reagent stabilized by the method.

### Background Art

Assaying various components contained in a biological sample, such as blood and urine, is essential for the diagnosis of disease, elucidation of pathological conditions, or assessment of therapeutic processes, because such components are thought to be implicated in some diseases. For example, there are methods that have been developed for assaying many varieties of minor components, such as blood cholesterol, triglyceride, glucose, uric acid, phospholipids, bile acid, and monoamine oxidase. These methods are of use in the diagnosis of some diseases.

Among the methods for assaying serum components is the enzymatic method in which an enzyme specifically acting on a target component is made active, and its resultant product is assayed for determination of the amount of the target component. This method is in widespread use. Among others, it is common to use a method in which an oxidase specifically acting on a target component is caused to act on the component, to thereby generate hydrogen peroxide, and subsequently, a color developing system is established by bringing the generated hydrogen peroxide to contact with an oxidizable color developing reagent (i.e., a reagent which develops color when oxidized), and peroxidase (POD), to thereby cause the reagent to develop color; and the amount of the target component is determined through colorimetric analysis of the thus-developed color. Examples of the oxidizable color developing reagents employed in such an enzymatic method include Trinder reagents, which is a phenolic, aniline, or toluidine chromogen, and forms a dye through oxidation-condensation with a coupler (e.g., 4-aminoantipyrine (4-AA) or 3-methyl-2-benzothiazolinonehydrazone (MBTH)) in the presence of POD. However, the color developing system associated with such an oxidizable color developing reagent has some disadvantages, such as its low sensitivity for quantification of minor components and its tendency to be affected by changes in absorption spectrum attributed to hemoglobin, bilirubin, etc. contained in a sample to be assayed. In recent years, many reports have been published in order to overcome such disadvantages, with regard to the oxidizable color developing reagents including a triphenylmethane leuco dye and a diphenylnaphthylmethane leuco dye, which directly develop color through oxidation in the presence of POD (see, for example, Patent Document 1). Further, triphenylmethane compounds are known to improve the low water-solubility of leuco dye (Patent Document 2). Leuco dyes have high sensitivities in assaying, and thus are very useful compounds for the quantification of minor compounds.

However, leuco dyes still have the problem that their storage stability is poor, thereby causing an unwanted nonspecific color development to occur with time.
Patent Document 1: JP-A-62-93261
Patent Document 2: JP-A-3-206896

### Disclosure of the Invention

### Problems to be Solved by the Invention

Accordingly, an object of the present invention is to provide a method for stably storing an oxidizable color developing reagent, in particular, a leuco dye. Another object of the present invention is to provide a reagent stabilized by such a method.

### Means for Solving the Problems

In view of the foregoing, the present inventors conducted extensive studies, and came to the finding that when an oxidizable color developing reagent is stored in a solution having a pH of 1 to 5, the oxidizable color developing reagent can be stably stored over a long period of time. Thus the present invention was accomplished on the basis of this finding.

Accordingly, the present invention provides a method for stabilizing an oxidizable color developing reagent, comprising storing the oxidizable color developing reagent in a solution having a pH of 1 to 5.
The present invention also provides an oxidizable color developing reagent solution having a pH of 1 to 5.

### Effect of the Invention

According to the stabilization method of the present invention, an oxidizable color developing reagent can be stably stored in a solution over a long period of time. Moreover, employment of the oxidizable color developing reagent solution of the present invention enables highly sensitive assay of a minor component of a biological sample. Therefore, the oxidizable color developing reagent solution of the present invention is very useful in the field of clinical examination.

### Best Mode for Carrying out the Invention

The oxidizable color developing reagent solution of the present invention may be employed in any oxidizing substance quantification method which employs an oxidizable color developing reagent as a color developing component. Examples of the oxidizing substance include hydrogen peroxide. The oxidizable color developing reagent solution of the present invention is particularly useful for the assay of minor components of a biological sample, in which an oxidase is caused to act on a substrate or a substance generated through enzymatic reaction, and the thus-generated hydrogen peroxide is quantified.

No particular limitation is imposed on the minor components contained in a biological sample and measurable through use of the oxidizable color developing reagent solution of the present invention. Thus, any biological component which can be assayed through quantification of hydrogen peroxide generated as a result of enzymatic reaction can become a measurement target of the present invention. Examples of such a component include glycated proteins, glycated peptides, glycated amino acids, cholesterol, glucose, glycerin, triglyceride, free fatty acids, uric acid, phospholipids, sialic acid, bile acid, pyruvic acid, inorganic phosphorus, creatinine, creatine, GOT, GPT, monoamine oxidase, guanase, and cholinesterase, etc.

No particular limitation is imposed on the oxidizable color developing reagent employable in the present invention. Examples of the reagent include a combination of 3-methyl-2-benzothiazolinonehydrazone (MBTH) and an aniline compound; 2,2'-azino-bis(3-ethylbenzthiazoline-6-sulphonic acid) (ABTS); leuco dyes; benzidine derivatives, o-tolidine derivatives, triallylimidazole derivatives, and o-phenylenediamine derivatives, etc. Of these reagents, leuco dyes are preferred.

Examples of the leuco dyes include triphenylmethane derivatives, phenothiazine derivatives, and diphenylamine derivatives, etc. Examples of the triphenylmethane derivatives which may be employed include highly watersoluble compounds described in JP-A-3-206896 and JP-A-6-197795, examples of the phenothiazine derivatives include the compounds described in JP-B-60-33479, and examples of the diphenylamine derivatives include the compounds described in JP-B-60-33479 and JP-A-62-93261. Of these compounds, preferred ones are Leuco Malachite green, Leuco Crystal Violet, N-(carboxymethyl-aminocarbonyl)-4,4'-bis(dimethylamino)-diphenylamine sodium salt (DA-64; Wako Pure Chemical Industries Ltd.), 10-(carboxymethyl-aminocarbonyl)-3,7-bis(dimethylamino)phenothiazine sodium salt (DA-67: Wako Pure Chemical Industries Ltd.), 10-(N-methylcarbamoyl)-3,7-bis(dimethylamino)-10H-phenothiazine (MCDP: product of Dojindo Laboratories), and N,N,N',N',N",N"-hexa-3-sulfopropyl-4,4',4"-triaminotriphenylmethane (TPM-PS: product of Dojindo Laboratories), etc. Of these dyes, TPM-PS, DA-64, DA-67, and MCDP are more preferred, with TPM-PS and MCDP being even more preferred.
Other employable dyes include diaminobenzidine, tetramethylbenzidine, hydroxyphenylpropionic acid, and orthophenylenediamine, etc.

In order to modify pH, any suitable substance may be used so long as it can attain an acidic pH. For example, there may be employed inorganic acids such as hydrochloric acid, sulfuric acid, and phosphoric acid; and organic acids such as glycine, phthalic acid, maleic acid, citric acid, succinic acid, oxalic acid, tartaric acid, acetic acid, and lactic acid. No particular limitation is imposed on the concentration of the inorganic or organic acids, but the concentration is preferably 0.0001 to 1,000 mM, particularly preferably 0.01 to 1,000 mM. The pH may be 1 to 5, but pH 1 to 4 is particularly preferred.

No particular limitation is imposed on the concentration of the oxidizable color developing reagent contained in an oxidizable color developing reagent solution. Preferably, the concentration is 0.001 to 100 mM, more preferably 0.001 to 50 mM.

The oxidizable color developing reagent solution of the present invention may also contain, for example, an anionic or nonionic surfactant each having a polyoxyethylene structure; an enzyme for treating contaminants in blood sample; a reaction-controlling agent; a stabilizer; a protein such as albumin; a salt such as sodium chloride, potassium chloride, or potassium ferrocyanide; an amino acid such as glycine, lysine, alanine, aspartic acid, or glutamic acid; a tetrazolium salt for preventing the effects of a reducing substance; an antibiotic; an antiseptic agent such as sodium azide or boric acid; or a cationic surfactant. The amount of such an additive may be appropriately determined with reference to known enzymatic quantification methods employing an oxidizable color developing reagent.

The oxidizable color developing reagent solution of the present invention may be provided as contained in, for example, a glass vial or a plastic container. Containers are preferably light shielded.

### Examples

The present invention will next be described in more detail with reference to examples, which should not construed as limiting the invention thereto.
[Example 1] Stabilization Test 1 for TPM-PS
TPM-PS was dissolved in each of the aqueous solutions described below so as to attain a concentration of 60 µM, and stored at 37°C. Subsequently, absorbance was measured at 600 nm by means of an automated analyzer (Model 7150; product of Hitachi, Ltd.). Table 1 shows the absorbance data as measured at points in time of 0 (start), 2-week storage, and 3-week storage.

**[Table 1]**

| Aqueous solution | Time 0 | 2-Week storage | 3-Week storage |
|---|---|---|---|
| 20mM PB-K (pH 8) | 0.023 | 0.132 | 0.215 |
| 5mM Tartaric acid (pH 2.8) | 0.024 | 0.064 | 0.088 |
| 5mM Maleic acid (pH 2.5) | 0.023 | 0.050 | 0.072 |
| 5mM Citric acid (pH 2.8) | 0.022 | 0.057 | 0.080 |

| | | | |
|---|---|---|---|
| *PB-K: Potassium phosphate solution | | | |

As apparent from Table 1, the nonspecific color development of TPM-PS was suppressed effectively in aqueous solutions of pH 1 to 5, indicating that TPM-PS is stable.

### Example 2 Stabilization Test 2 for TPM-PS

TPM-PS was dissolved in each of the aqueous solutions described below so as to attain a concentration of 100 µM, and absorbance was measured at 600 nm. After each sample was stored at 25°C for 10 days, absorbance was again measured at 600 nm. Table 2 shows the difference between the absorbance measured immediately after preparation of the sample and that measured after 10 days of storage (referred to as "variation in 10 days (OD)").

**[Table 2]**

| | pH of Stored solution | Variation over 10 days (OD) |
|---|---|---|
| HCl-KCl | 1 | -0.01 |
| | 2 | 0.01 |
| 100mM Glycine-HCl | 2 | 0.00 |
| | 3 | -0.01 |
| 100mM Citrate buffer | 3 | -0.01 |
| | 4 | 0.08 |
| | 5 | 0.15 |
| 100mM Potassium phosphate buffer | 6 | 0.22 |
| | 7 | 0.16 |
| | 8 | 0.17 |
| Control (purified water) | Not regulated | 0.22 |

As apparent from Table 2, the absorbance variation of TPM-PS stays in a small range when stored in aqueous solution of pH 1 to 5, indicating that TPM-PS is stably stored as a result.

### Example 3 Stability of MCDP

MCDP was dissolved in methanol so as to attain a concentration of 4 mM, and the resultant MCDP-methanol was dissolved in each of the below-described aqueous solutions supplemented with 0.1% Triton X-100, so as to attain a concentration of 100 µM. The solutions were stored at 37°C for 24 hours. Subsequently, absorbance was measured at 600 nm. The results are shown in Table 3.

**[Table 3]**

| | pH of Stored solution | Variation over 24 hrs (OD) |
|---|---|---|
| HCl-KCl | 1 | 0.01 |
| | 2 | 0.01 |
| 50mM Glycine-HCl | 2 | 0.00 |
| | 3 | 0.00 |
| 50mM Citrate buffer | 3 | 0.01 |
| | 4 | 0.06 |
| | 5 | 0.12 |
| 50mM Potassium phosphate buffer | 6 | 0.83 |
| | 7 | 1.47 |
| | 8 | 1.51 |
| Control (purified water) | Not regulated | 0.71 |

As apparent from Table 3, the absorbance variation of MCDP is too small, and the nonspecific color development of MCDP is effectively suppressed when stored in aqueous solution of pH 1 to 5, indicating that MCDP can be stored stably.

## Claims

1. A method for stabilizing an oxidizable color developing reagent, comprising storing an oxidizable color developing reagent in a solution of pH 1 to 5.

2. The method according to claim 1, wherein the oxidizable color developing reagent is a leuco dye selected from a triphenylmethane leuco dye, a phenothiazine leuco dye, and a diphenylamine leuco dye.

3. The method according to claim 2, wherein the triphenylme thane leuco dye is N,N,N',N',N",N"-hexa-3-sulfopropyl-4,4', 4"-triaminotriphenylmethane.

4. The method according to claim 2, wherein the phenothiazine leuco dye is 10-(N-methylcarbamoyl)-3,7-bis(dimethylamino)-10H-phenothiazine or 10-(carboxymethylaminocarbonyl)-3,7-bis(dimethylamino)phenothiazine.

5. The method according to any one of claims 1 to 4, wherein the solution of pH 1 to 5 contains one or more species selected from among hydrochloric acid, sulfuric acid, phosphoric acid, and organic acids.

6. The method according to claim 5, wherein the organic acid is maleic acid or citric acid.

7. A solution of an oxidizable color developing reagent, wherein the solution has a pH of 1 to 5.
